# FASCICULE DE BREVET EUROPEEN

(11) **EP 3 596 045 B1**
(45) Date de publication et mention de la délivrance du brevet: **28.04.2021**
(21) Numéro de dépôt: 18713322.8
(22) Date de dépôt: 09.03.2018
(51) Int. Cl.: C07C 319/28, C07C 323/52

(54) **PROCÉDÉ DE FABRICATION DE L'ACIDE-2-HYDROXY-4-MÉTHYLTHIO-BUTYRIQUE**
VERFAHREN ZUR HERSTELLUNG VON 2-HYDROXY-4-METHYLTHIO-BUTANSAÜRE
PROCESS FOR THE PREPARATION OF 2-HYDROXY-4-METHYLTHIO-BUTYRIC ACID

(30) Priorité: 16.03.2017 FR 1752163
(43) Date de publication de la demande: 22.01.2020
(73) Titulaire: Adisseo France S.A.S., 92160 Antony (FR)
(72) Inventeur: BELLIERE-BACA, Virginie, 69390 Millery (FR); MORVAN, Didier, 69440 Mornant (FR)
(74) Mandataire: Agasse, Stéphane
(86) Numéro de dépôt international: PCT/FR2018/050550
(87) Numéro de publication internationale: WO 2018/167405

(56) Documents cités:
- US-A- 4 912 257

## Description

La présente invention concerne un procédé amélioré de préparation de l'acide 2-hydroxy-4-méthylthio-butyrique (HMTBA) et son homologue sélénié, l'acide 2-hydroxy-4-méthylséléno-butyrique. Plus particulièrement, ce procédé intègre une étape performante de purification de l'HMTBA obtenu par hydrolyse acide du 2-hydroxy-4-méthylthio-butyronitrile (HMTBN).

L'acide 2-hydroxy-4-méthylthio-butyrique (HMTBA) et ses analogues tels que les sels, les chélates, notamment les chélates métalliques (de Zn, Ca, Mn, Mg, Cu, Na...) et les esters de ces acides, comme les esters isopropylique et tertiobutylique de l'HMTBA, sont largement utilisés en nutrition animale. Les dérivés séléniés de ces hydroxyanalogues de la méthionine sont eux aussi des constituants d'intérêts majeurs en nutrition animale.

La préparation d'HMTBA peut être opérée par différents procédés d'hydrolyse directe ou indirecte, de l'HMTBN. Cette hydrolyse est classiquement réalisée par un acide minéral tel que l'acide chlorhydrique ou l'acide sulfurique, elle peut aussi être réalisée par hydrolyse enzymatique.

Ainsi WO 00/02852A1 décrit la fabrication d'HMTBA par hydrolyse d'HMTBN avec de l'acide sulfurique en deux étapes. Selon une première étape [1], on réalise une réaction d'hydratation du 2-hydroxy-4-méthylthio-butyronitrile (HMTBN) en 2-hydroxy-4-méthylthio-butyramide (HMTBM) qui est ensuite hydrolysé dans une seconde étape [2] en acide 2-hydroxy-4-méthylthio-butyrique (HMTBA), comme représenté par les réactions ci-dessous :

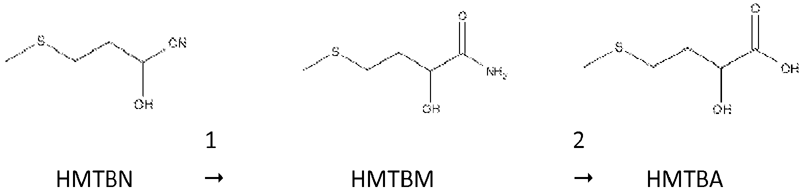

Le mélange obtenu contenant l'HMTBA peut être ensuite traité par une ou plusieurs étapes de purification comme décrit dans les documents WO 00/02853A1, US 4524077A, US 4912257A ou JP 2007238555A.

Ainsi selon US 4524077A, on réalise une extraction directe du milieu d'hydrolyse par un solvant non miscible à l'eau, suivie d'une évaporation dudit solvant en présence d'une quantité d'eau de façon à réduire l'apparition d'une coloration brune du produit obtenu. Le solvant est choisi notamment parmi la méthyléthylcétone, la méthylisobutylcétone, le méthyltertiobutyléther, le diisopropyléther, le diéthylcarbonate. On observe alors le relargage d'une phase aqueuse saline. Le solvant est éliminé de la phase organique par évaporation et la solution finale d'HMTBA est adaptée au titre commercial par addition d'eau.

US 4912257A décrit, à la suite des étapes d'hydrolyse d'HMTBN en présence d'acide sulfurique et de neutralisation du milieu réactionnel par de l'ammoniaque, une étape de séparation d'une phase organique contenant l'HMTBA et des sels subsistants et d'une phase aqueuse contenant des sels, essentiellement du sulfate d'ammonium et des traces d'HMTBA, la phase organique étant ensuite concentrée puis filtrée pour récupérer l'HMTBA, d'une part, et la phase aqueuse étant concentrée puis les sels précipités, d'autre part. La mise au titre final de l'HMTBA ainsi obtenu est effectuée par addition d'eau.

Enfin le document JP 2007238555A propose de réaliser, après l'étape d'hydrolyse mais avant l'étape de neutralisation, une distillation pour retirer des molécules légères, majoritairement soufrées et l'acide formique. Cette distillation s'opère à des températures entre 80°C et 120°C et à des pressions entre 0,5 et 1,5 bar. Une séparation biphasique en deux phases organique et aqueuse est ensuite effectuée comme dans les documents précédents et les deux phases aussi traitées de la même manière.

La synthèse de l'acide 2-hydroxy-4-méthylséléno-butyrique est elle aussi connue. Il peut notamment être fabriqué par hydrolyse acide du 2-hydroxy-4-méthylséléno-butyronitrile comme décrit dans le document WO 2008/049927A1.

Quelle que soit la technique de séparation des acides précités des sels résultant de la neutralisation, ceux-ci, et spécifiquement le sulfate d'ammonium, peuvent être valorisés et en particulier commercialisés tels quels ou après un ou des traitements complémentaires de purification.

Les procédés connus tels que ceux présentés ci-dessus souffrent toutefois d'inconvénients multiples résultant de la technique d'isolement du produit final HMTBA. Ils sont résumés ci-après :
a) La présence inévitable de 1 à 2% poids de sulfate d'ammonium, affectant la qualité de l'HMTBA et qui dans le produit fini, se substitue à l'eau. En effet, cette plus faible présence d'eau induit, par des lois thermodynamique d'équilibre, une plus grande concentration d'oligomères de l'HMTBA dans le produit fini. Ceci engendre une plus grande viscosité du produit fini et limite son acidité ;
b) Une couleur brune foncée et une mauvaise odeur ;
c) Coût opératoire élevé en raison de la fiabilité et de la maintenance des étapes de filtration ;
d) Coût élevé du produit final en raison de l'emploi de moyens tels que l'extraction avec des solvants.

Le procédé de l'invention permet de surmonter l'ensemble de ces inconvénients grâce à la mise en œuvre d'une étape d'isolement de l'HMTBA qui peut se substituer à celle(s) existante(s), cet isolement étant réalisé par chromatographie d'un flux d'HMTBA issu du milieu réactionnel d'hydrolyse et neutralisation.

Ainsi, l'invention apporte un procédé de fabrication de l'HMTBA à partir de HMTBN, comprenant les étapes suivantes :
On hydrolyse l'HMTBN en HMTBA en présence d'un acide minéral en milieu aqueux,
On neutralise ledit milieu par ajout d'une base,
On sépare une première phase comprenant au moins l'HMTBA et des sels et une seconde phase contenant des sels,
ledit procédé comprenant une étape selon laquelle on réalise la séparation de l'HMTBA desdits sels de la première phase, en soumettant cette dernière à une chromatographie.

Par chromatographie selon l'invention, on entend toute méthode séparative qui permet de séparer une phase comprenant au moins de l'HMTBA et des sels en deux phases, l'une enrichie en HMTBA et l'autre enrichie en sels. Une telle méthode met en œuvre une phase stationnaire et une phase mobile. A titre d'exemples, elle peut être réalisée en lit statique ou non statique dans une ou plusieurs colonnes tels que les systèmes de chromatographie d'au moins deux colonnes décrits dans FR2889077 ou dans l'article Separation Science and Technology 35(4):519-534, 2000), ou les systèmes de chromatographie d'au moins trois colonnes comme les technologies iSMB telle que décrite dans EP 0342629 et US 5064539), SSMB, AMB, VARICOL™ (telle que décrite dans US 6,136,198, US 6,375,839, US 6,413,419 and US 6,712,973), MODICON™ (telle que décrite dans US 7,479,228), POWERFEED™ (telle que décrite dans US 5,102,553 et l'article «Power Feed operation of simulated moving bed units: changing flow-rates during the switching interval», Zhang et al. in Journal of Chromatography A, 1006:87-99, 2003), ou MCSGP (Multicolumn Countercurrent Solvent Gradient Purification).

Il est apparu qu'il est possible d'éliminer des sels de façon très efficace d'un flux contenant de l'HMTBA par une chromatographie qui fait intervenir un double effet d'exclusion à la fois stérique et ionique. A titre indicatif, l'utilisation de cette étape de chromatographie permet de séparer 95% du sulfate d'ammonium contenu dans la première phase ainsi traitée.

La chromatographie est avantageusement réalisée par traitement de la première phase contenant l'HMTBA sur une résine. La résine peut être une résine anionique ou cationique.

Si la résine est anionique, elle est de préférence chargée en anions choisis parmi OH⁻, Cl⁻, SO₄²⁻.

Si elle est cationique, elle est de préférence chargée en cations choisis parmi NH₄⁺, H⁺, Na⁺, K⁺ et Ca²⁺.

Avantageusement, la chromatographie est effectuée selon un mode en lit mobile séquentiel simulé ou Sequential Simulated Moving Bed (SSMB). Par différence d'affinité entre la phase liquide consistant en une solution aqueuse de HMTBA et le solide de la colonne, cette technique permet de séparer les sels minéraux, ici le sulfate d'ammonium, des espèces organiques, ici majoritairement l'HMTBA. En plus de son efficacité, cette séparation ne nécessite aucun ajout d'autre réactif et n'entraîne la formation d'aucun sel supplémentaire. Un autre avantage à cette technologie est la facilité d'exploitation et la longue durée de vie des colonnes.

Le flux d'HMTBA qui est traité dans le cadre du procédé de l'invention est donc issu du milieu de neutralisation. Selon une variante, l'étape de neutralisation peut être précédée d'une étape de distillation telle que mentionnée précédemment en référence au document JP 2007238555A, et en particulier dans des conditions de température de 80°C à 120°C et de pression de 0,5 à 1,5 bar. Le flux d'HMTBA traité contient majoritairement de l'HMTBA. Il peut contenir de l'eau, des sels, des impuretés et des sous-produits, notamment organiques comme des hydroxybutyrolactones fonctionnalisées et de l'aldéhyde méthylthioproprionique (MMP). De préférence, la concentration en HMTBA du flux à traiter par chromatographie est de 30 à 90% (m/m), voire de 50 à 80% (m/m). Si nécessaire, elle est ajustée par addition d'eau.

Dans une version préférée, l'éluant de chromatographie est un solvant aqueux. Il est avantageusement choisi parmi l'eau, l'eau étant pure ou issue d'un recyclage, et ses mélanges avec un ou des solvants organiques. Ces derniers sont de préférence choisis parmi les alcools, comme le méthanol et l'éthanol, les furanes comme le tétrahydrofurane, et l'acétonitrile.

Selon une variante de l'invention, le solvant aqueux est choisi parmi l'eau, une solution aqueuse acide et une solution aqueuse basique. Il est du ressort des compétences et connaissances générales de l'homme du métier de choisir l'acide ou la base, ainsi que leur concentration, en fonction du flux d'HMTBA, étant entendu que dans une mise en œuvre à l'échelle industrielle, les solutions les plus économiques seront retenues.

Avantageusement, la chromatographie est réalisée dans des conditions de vitesse d'élution que l'homme du métier déterminera au vu de ses connaissances générales.

Dans une mise en œuvre particulière de l'étape de séparation, la chromatographie est effectuée selon un mode en lit simulé (SMB) ou selon un mode en lit mobile séquentiel simulé (SSMB).

Bien que l'étape de séparation décrite ci-dessus soit adaptée à tout flux d'HMTBA, dans un mode préféré, il est séparé des sels de sulfate d'ammonium NH₄HSO₄ ou (NH₄)₂HSO₄ et leurs mélanges, provenant d'une hydrolyse de l'HMTBA en présence d'acide sulfurique puis neutralisation par ajout d'ammoniac ou d'hydroxyde d'ammonium.

Toute étape supplémentaire aux fins de la purification de l'HMTBA peut être incorporée dans le procédé à tout stade de celui-ci. Ainsi, comme indiqué précédemment, une étape de distillation du milieu d'hydrolyse, pour le stripping des molécules légères, avant neutralisation, peut être prévue. Aussi, selon une mise en œuvre très avantageuse du procédé de l'invention, l'étape de séparation par chromatographie précitée est réalisée sur un flux d'HMTBA qui est issu d'une étape de séparation, par décantation, de la première phase comprenant au moins l'HMTBA et des sels et de la seconde phase contenant des sels. L'efficacité de séparation est dans ces conditions beaucoup plus grande et la durée de vie des colonnes de chromatographie considérablement augmentée. Toute autre technique de séparation de ces deux phases telle que centrifugation ou séparation liquide/liquide peut être employée dès lors qu'elle n'implique pas d'extraction avec des solvants. Elle facilite extraordinairement les processus subséquents de purification et de récupération totale du HMTBA, d'une part, et du sulfate d'ammonium, d'autre part, avec un rendement et une efficacité élevés. Ainsi des techniques de séparation du type extraction impliquant un solvant non miscible à l'eau ne sont pas nécessaires voire contre-productives ; elles n'améliorent pas substantiellement la qualité ni le rendement, augmentent le prix du procédé et compliquent l'installation.

A la sortie de l'étape de séparation par chromatographie, on récupère une phase riche en HMTBA et une phase riche en sels. L'une et/ou l'autre peuvent être soumises à évaporation et le solvant aqueux résultant recyclé à au moins l'une quelconque des étapes dudit procédé.

En sortie de colonne de chromatographie, l'HMTBA est ensuite remis au titre par évaporation de l'excédent d'eau apporté lors de la séparation sur ladite colonne. Cette amélioration du procédé de fabrication de l'HMTBA permet d'augmenter la qualité du produit fini ainsi que faciliter l'exploitation.

Le produit obtenu est un HMTBA dilué qui sera remis à un titre pouvant atteindre 95%, lors d'une étape d'évaporation. L'eau retirée de l'HMTBA sera avantageusement réintroduite dans le procédé à l'étape de séparation chromatographique ou ailleurs. La phase contenant le sulfate d'ammonium pourra être réintroduite à l'étape de neutralisation ou ailleurs.

Le reste du procédé restant identique.

Cette séparation pourra également être utilisée pour séparer avantageusement les espèces soufrées autres que l'HMTBA pour améliorer la qualité du produit fini. Les phases contenant l'HMTBA et le sulfate d'ammonium seront traitées de la même façon que décrit plus haut, la nouvelle phase contenant des produits soufrés sera alors séparée et traitée à convenance.

L'invention concerne aussi l'acide 2-hydroxy-4-méthylthio-butyrique obtenu selon le procédé ci-dessus, dans lequel le taux d'oligomères est réduit par rapport à l'HMTBA obtenu selon un procédé de l'art antérieur et dont la proportion en sels est d'au plus 0,8% (m/m), voire d'au plus 0,5% et même d'au plus 0,3%, et même des sels seulement à l'état de traces.

La présente invention sera plus complètement décrite et ses avantages par rapport à l'état de la technique ressortiront à l'aide des exemples illustrant la fabrication d'HMTBA selon un procédé de l'invention, à l'échelle industrielle, et à l'appui des figures 1-4 ci-jointes selon lesquelles :
La figure 1 est un schéma d'un procédé industriel connu de fabrication de l'HMTBA;
La figure 2 est un schéma d'un procédé de fabrication de l'HMTBA, selon l'invention, à l'échelle industrielle ;
La figure 3 est une représentation graphique de la séparation entre le sulfate d'ammonium et l'HMTBA
La figure 4 est une représentation graphique de la séparation d'hydroxybutyrolactones fonctionnalisées.

Cet exemple n'est bien entendu pas limitatif tant dans les conditions de mises en œuvre que dans le composé obtenu, qui selon l'invention peut aussi être l'acide 2-hydroxy-4-méthylséléno-butyrique.

### EXEMPLE : Fabrication d'HMTBA à partir d'HMTBN, selon l'invention

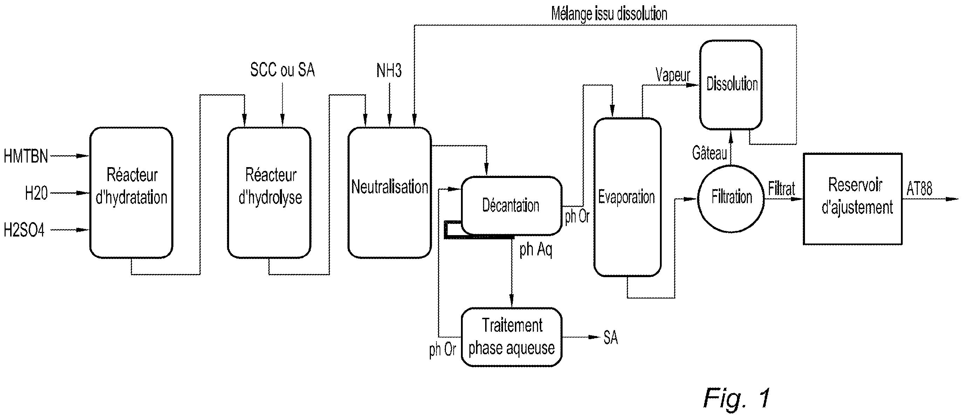

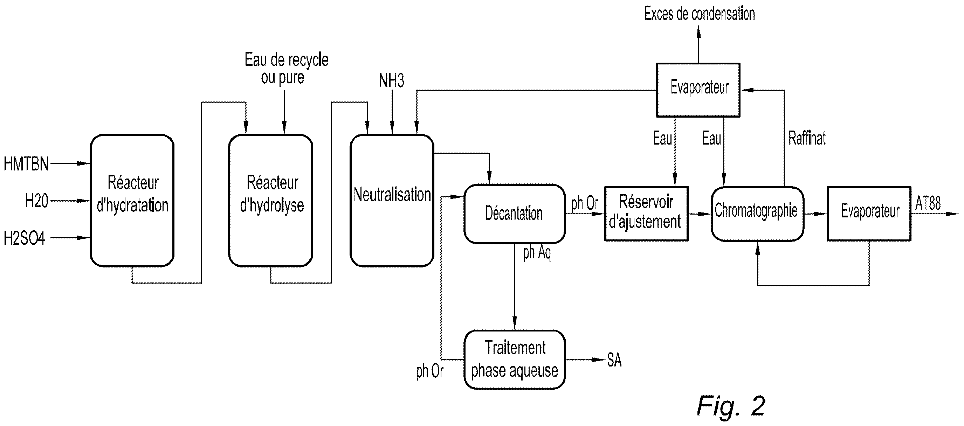

Le procédé suivi est illustré à la figure 2. Il peut être comparé à la figure 1 correspondant à un procédé classique de synthèse de l'HMTBA.

### 1) Synthèse de l'HMTBN

L'HMTBN est synthétisé selon un procédé qui est une variante de celui exposé dans le document EP 0739870A1 décrit pour la synthèse de l'acide aminé DL-méthionine. Il n'en diffère que par le réactif de synthèse du nitrile, qui selon le présent exemple est de l'eau exempte d'ammoniac, alors que selon le document, le réactif est une solution aqueuse ammoniacale. Cette fabrication est bien connue de l'homme du métier.

### 2) Synthèse de l'HMTBA

### 2.1) Hydrolyse de l'HMTBN

Le produit issu de l'étape de synthèse ci-dessus est mis en contact d'acide sulfurique concentré, de préférence à 98 %, dans une boucle d'acidification où le produit et l'acide sont mélangés les deux produits. Afin d'éviter des échauffements locaux conduisant à la destruction du nitrile et à la formation de réactions secondaires entraînant une augmentation de coloration, l'acidification est réalisée en continu avec une grande recirculation, en ajoutant l'acide sulfurique concentré à la solution acidifiée du nitrile, cette solution ayant de préférence une concentration de 20-50 % en poids d'acide.

Comme il est nécessaire d'éliminer la chaleur de dilution de l'acide sulfurique, la boucle d'acidification est pourvue d'un ou de plusieurs échangeurs de chaleur de façon que la température de réaction ne dépasse pas 65°C.

Le rapport molaire acide sulfurique/HMTBN est compris entre 0,8-1,5. La proportion d'eau est ajustée de telle manière que la solution acidifiée soit constituée par une seule phase et soit capable de maintenir en solution le sulfate d'ammonium qui va se former durant la réaction d'hydrolyse et au cours de la neutralisation avec de l'hydroxyde d'ammonium qui suit.

Après un temps de contact de 30-60 minutes, de l'eau est ajoutée jusqu'à obtenir une concentration de 20% à la sortie de l'étape d'hydrolyse. Le milieu réactionnel obtenu est chauffé à une température comprise entre 110 et 130°C, le temps de séjour est compris entre 2 heures et 4 heures pour obtenir le HMTBA.

Pendant le déroulement de l'hydrolyse, il convient d'appliquer au réacteur un léger vide (entre environ 20 et 200 mm) de façon à éliminer le petit excès de HCN utilisé dans la synthèse du HMTBN, ainsi que les impuretés volatiles qui pourraient s'être formées dans la réaction et auxquelles on attribue la mauvaise odeur du produit final.

### 2.2) Neutralisation du milieu d'hydrolyse

Le mélange réactionnel d'hydrolyse est refroidi à 50-70°C, et son excès d'acide est neutralisé avec une solution d'ammoniac de 20-35% en poids, ladite solution pouvant éventuellement être formée in situ par barbotage de NH₃ gazeux. Ladite neutralisation peut nécessiter un refroidissement pour ne pas dépasser la température de 90°C.

### 3) Purification de l'HMTBA

### 3.1) Etapes préalables de séparation

La masse neutralisée obtenue comprend deux phases qui présentent des densités nettement différentes et peuvent être facilement décantées Elles consistent en une première phase qui contient 93-95 % de l'HMTBA formé et une seconde phase qui renferme le reste. Le sulfate d'ammonium formé durant la réaction d'hydrolyse et la neutralisation de l'acide utilisé en excès, se répartit entre les deux phases, la seconde phase étant la plus riche avec 70-75 % en poids.

A partir de la seconde phase on précipite le sulfate d'ammonium par évaporation de l'eau à la pression atmosphérique ou sous pression réduite. Le solide résultant est séparé par n'importe quel procédé standard de séparation solide-liquide tel que la filtration et/ou la centrifugation, et le liquide ainsi obtenu, contenant la partie du sulfate d'ammonium qui n'a pas précipité et le HMTBA, est recyclé au récipient de neutralisation. Ce procédé permet d'obtenir du sulfate d'ammonium qui, une fois séché, possède une grande pureté et est pratiquement exempt de HMTBA ; ce dernier est récupéré dans sa totalité par recyclage en tête du processus de séparation, tout en restant incorporé à la première phase.

### 3.2) Etape de séparation par chromatographie selon l'invention

La première phase qui provient du décanteur et contient l'HMTBA des deux flux (de neutralisation et de recyclage) est conduite à un réservoir d'ajustement pour effectuer une dilution avant purification sur colonne chromatographique. En fonctionnement normal, 800t par jour de première phase sont traitées et dans le cadre de l'invention cette phase est diluée avec 240t par jour d'eau de recyclage provenant de l'évaporation post-purification sur colonne chromatographique. Cette première phase contient notamment et avant dilution, 65%p/p d'HMTBA, 14%p/p de sulfate d'ammonium majoritairement sous sa forme (NH₄)₂SO₄.

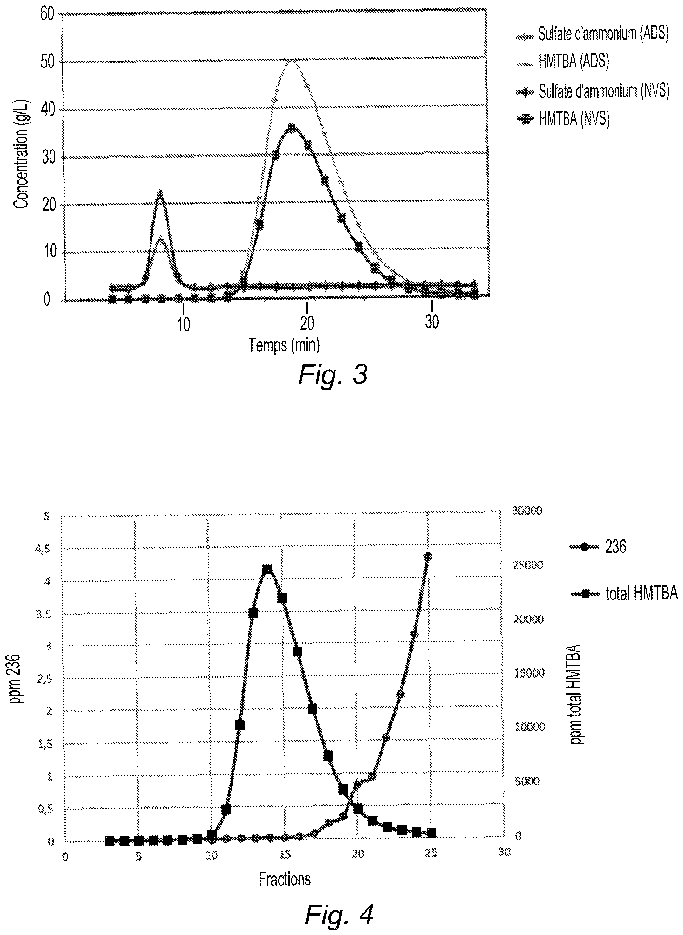

La première phase diluée est ensuite acheminée sur une colonne chromatographique de technologie lit mobile simulé pour purification. L'éluant utilisé est de l'eau de recyclage provenant de l'évaporation post-purification. 3700t par jour d'eau sont ainsi utilisées pour purification de la première phase qui représente 1040t par jour après l'étape de dilution. La séparation se fait dans la colonne par exclusion stérique et ionique entre la phase liquide et la phase solide de la colonne. La séparation entre le sulfate d'ammonium et l'HMTBA est représenté sur la figure 3, sur laquelle l'HMTBA est représenté sous l'appellation « *Organic* » et le sulfate d'ammonium sous l'appellation « *Salt* »*.* Les sigles ADS et NVS représentant des analyses par chromatographie liquide effectuées sur deux sites différents pour confirmation des résultats

La séparation entre le sulfate d'ammonium et l'HMTBA est de 95%. Autrement dit, 95% du sulfate d'ammonium contenu dans la première phase sont séparés par la technologie de lit mobile simulé.

Cette séparation sur colonne chromatographique permet la possibilité, en outre, de séparer une impureté majoritaire organique contenue dans la première phase. Cette impureté, appelée MW236, est une hydroxybutyrolactone fonctionnalisée. La représentation graphique de cette séparation est donnée sur la figure 4.

80%p/p de cette impureté peut être retirée de la première phase par cette technologie séparative.

Il ressort de cet exemple que l'étape de purification par chromatographie peut remplacer au moins l'une voire toutes les étapes de séparation classiquement effectuées, comme la comparaison des figures 1 et 2 le met en évidence, et qu'elle permet d'obtenir un HMTBA, quasiment exempt de sels, et diminué en oligomères et en impuretés notamment organiques comme les hydroxybutyrolactones fonctionnalisées et le MMP.

La séparation des espèces ne se limitent pas à ces exemples.

Les exemples suivants illustrent l'étape de purification du procédé de l'invention réalisée sur d'autres flux d'HMTBA que celui de l'exemple précédent.

A la sortie de la colonne de chromatographie deux flux principaux sont obtenus, un premier représentant 1140t par jour et contenant 95% du sulfate d'ammonium contenu dans la première phase de départ et un deuxième représentant 3600t par jour d'une phase riche en HMTBA. La phase riche en sulfate d'ammonium est appelée raffinat. Les deux phases sont ensuite acheminées vers des évaporateurs pour concentration et récupération de l'eau pour recyclage.

La phase riche en sulfate d'ammonium, appelée raffinat, est acheminée vers un évaporateur pour concentration et récupération de l'eau pour recirculation dans le procédé. Un évaporateur par compression mécanique est utilisé mais toute technologie d'évaporateur connue de l'homme de l'art peut être utilisée avantageusement à cette étape du procédé.

L'eau récupérée à cette étape est redistribuée à deux endroits du procédé. Un premier flux représentant 655t par jour d'eau est recyclé à la chromatographie et utilisé comme éluant et un second représentant 240t par jour d'eau, utilisé dans l'étape de dilution de la première phase.

La phase concentrée en sulfate d'ammonium qui représente 125t par jour est recyclée à l'étape de neutralisation du procédé.

Un excès de condensat, représentant 105t par jour est éliminé du système à cette étape.

La phase riche en HMTBA sortie de l'étape de chromatographie est elle aussi condensée par évaporateur à compression mécanique ou toute technologie appropriée d'évaporation connue de l'homme de l'art. L'eau récupérée par évaporation est recyclée dans le procédé à l'étape de chromatographie et sert comme éluant. Cette eau représente 3020t par jour.

Cette évaporation permet également d'obtenir une phase riche en HMTBA qui est avantageusement concentrée à 88% p/p d'HMTBA pour obtenir directement le produit fini, appelée AT88. Ce flux de produit AT88 représente 560t par jour. Typiquement ce produit fini contient (en p/p): 88% d'HMTBA et ses dérivés oligomériques, 11,2% d'eau, 0,8% de sulfate d'ammonium et des traces d'autres produits organiques.

Le tableau ci-après présente l'efficacité d'un procédé de l'invention par rapport à un procédé connu, à partir d'un même flux de la première phase en sortie de décantation à un débit de 33 300 kg/h, en donnant la composition du produit fini :

**Tableau**

| % | Procédé de l'invention | Procédé connu |
|---|---|---|
| HMTBA | 88 | 88 |
| Eau | 11,2 | 10 |
| (NH₄)₂SO₄ | 0,8 | 1,6 |
| Hydroxybutyrolactones fonctionnalisées | 0 | 0,5 |
| MMP | 0 | 0,08 |

Grâce au procédé de l'invention, on obtient l'HMTBA dans lequel la proportion subsistante de sels est diminuée de moitié et dans lequel on ne détecte plus d'impuretés organiques.

## Revendications

1. Procédé de fabrication de l'acide-2-hydroxy-4-méthylthio-butyrique (HMTBA) à partir du 2-hydroxy-4-méthylthio-butyronitrile (HMTBN), comprenant les étapes suivantes :
On hydrolyse l'HMTBN en HMTBA en présence d'un acide minéral en milieu aqueux,
On neutralise ledit milieu par ajout d'une base,
On sépare une première phase comprenant au moins l'HMTBA et des sels et une seconde phase contenant des sels,
Ledit procédé étant **caractérisé en ce qu'**on réalise la séparation de l'HMTBA desdits sels de la première phase, en soumettant cette dernière à une chromatographie .

2. Procédé selon la revendication 1, caractérisé ce que la chromatographie est effectuée par traitement de la première phase contenant l'HMTBA sur une résine.

3. Procédé selon la revendication 2, **caractérisé en ce que** la résine est une résine anionique qui est de préférence chargée en anions choisis parmi OH⁻, Cl⁻, SO₄²⁻.

4. Procédé selon la revendication 2, **caractérisé en ce que** la résine est une résine cationique qui est de préférence chargée en cations choisis parmi NH₄⁺, H⁺, Na⁺, K⁺ et Ca²⁺.

5. Procédé selon l'une quelconque des revendications précédentes, **caractérisé en ce que** l'éluant de la chromatographie est un solvant aqueux.

6. Procédé selon la revendication 5, **caractérisé en ce que** le solvant aqueux est choisi parmi l'eau et ses mélanges avec un ou des solvants organiques tels que les alcools et notamment le méthanol et l'éthanol, les furanes et notamment le tétrahydrofurane et l'acétonitrile.

7. Procédé selon la revendication 5 ou 6, **caractérisé en ce que** le solvant aqueux est choisi parmi l'eau, une solution aqueuse acide et une solution aqueuse basique.

8. Procédé selon l'une quelconque des revendications précédentes, **caractérisé en ce que** la chromatographie est effectuée selon un mode en lit simulé (SMB) ou selon un mode en lit mobile séquentiel simulé (SSMB).

9. Procédé selon l'une quelconque des revendications précédentes, caractérisé ce que, avant séparation, la concentration de la première phase en HMTBA va de 30 à 90% (m/m), de préférence 50 à 80% (m/m).

10. Procédé selon la revendication 9, **caractérisé en ce que** la concentration en HMTBA est ajustée par addition d'eau.

11. Procédé selon l'une quelconque des revendications précédentes, **caractérisé en ce que** l'hydrolyse de l'HMTBN est effectuée en présence d'acide sulfurique et **en ce que** la neutralisation est effectuée par ajout d'ammoniac ou d'hydroxyde d'ammonium.

12. Procédé selon l'une quelconque des revendications précédentes, **caractérisé en ce que**, après l'hydrolyse de l'HMTBN et avant la neutralisation, on réalise une distillation à une température de 80°C à 120°C et une pression de 0,5 à 1,5 bar.

13. Procédé selon la revendication 1, caractérisé ce qu'on sépare la première phase comprenant au moins l'HMTBA et des sels et la seconde phase contenant des sels, par décantation.

14. Procédé selon l'une quelconque des revendications 1 à 13, **caractérisé en ce que** la phase riche en HMTBA et/ou la phase riche en sels récupérées à l'issue de la chromatographie sont soumises à évaporation et le solvant aqueux résultant recyclé à au moins l'une quelconque des étapes dudit procédé.

## Patentansprüche

1. Verfahren zur Herstellung von 2-Hydroxy-4-(methylthio)buttersäure (HMTBA) aus 2-Hydroxy-4-(methylthio)buttersäurenitril (HMTBN), umfassend die folgenden Schritte:
Hydrolysieren der HMTBN in HMTBA in Anwesenheit einer mineralischen Säure in wässrigem Milieu,
Neutralisieren des Milieus durch Hinzufügen einer Base,
Separieren einer ersten Phase, die mindestens HMTBA und Salze umfasst und einer zweiten Phase, die Salze enthält,
wobei das Verfahren **dadurch gekennzeichnet ist, dass** das Separieren der HMTBA von den Salzen der ersten Phase durch Chromatographie durchgeführt wird.

2. Verfahren nach Anspruch 1, **dadurch gekennzeichnet, dass** die Chromatographie durch Behandeln der ersten Phase, die die HMTBA enthält, auf einem Harz durchgeführt wird.

3. Verfahren nach Anspruch 2, **dadurch gekennzeichnet, dass** das Harz ein anionisches Harz ist, das vorzugsweise mit Anionen geladen ist, die aus OH⁻, Cl⁻, SO₄²⁻ ausgewählt sind.

4. Verfahren nach Anspruch 2, **dadurch gekennzeichnet, dass** das Harz ein kationisches Harz ist, das vorzugsweise mit Kationen geladen ist, die aus NH₄⁺, H⁺, Na⁺, K⁺ und Ca²⁺ ausgewählt sind.

5. Verfahren nach einem der vorangehenden Ansprüche, **dadurch gekennzeichnet, dass** das Eluat der Chromatographie ein wässriges Lösungsmittel ist.

6. Verfahren nach Anspruch 5, **dadurch gekennzeichnet, dass** das wässrige Lösungsmittel aus dem Wasser und seinen Gemischen mit einem oder mit organischen Lösungsmitteln wie den Alkoholen und insbesondere dem Methanol und dem Ethanol, den Furanen und insbesondere dem Tetrahydrofuran und dem Acetonitril ausgewählt ist.

7. Verfahren nach Anspruch 5 oder 6, **dadurch gekennzeichnet, dass** das wässrige Lösungsmittel aus dem Wasser, einer sauren wässrigen Lösung und einer basischen wässrigen Lösung ausgewählt ist.

8. Verfahren nach einem der vorangehenden Ansprüche, **dadurch gekennzeichnet, dass** die Chromatographie gemäß einem Simulated Moving Bed-Modus (SMB) oder gemäß einem Smart Simulated Moving Bed-Modus (SSMB) durchgeführt wird.

9. Verfahren nach einem der vorangehenden Ansprüche, **dadurch gekennzeichnet, dass** vor dem Separieren die Konzentration der ersten Phase an HMTBA von 30 bis 90 % (m/m), vorzugsweise 50 bis 80 % (m/m), beträgt.

10. Verfahren nach Anspruch 9, **dadurch gekennzeichnet, dass** die Konzentration an HMTBA durch Hinzufügen von Wasser eingestellt wird.

11. Verfahren nach einem der vorangehenden Ansprüche, **dadurch gekennzeichnet, dass** das Hydrolysieren der HMTBN in Anwesenheit von Schwefelsäure durchgeführt wird und dass das Neutralisieren durch das Hinzufügen von Ammoniak oder Ammoniumhydroxid durchgeführt wird.

12. Verfahren nach einem der vorangehenden Ansprüche, **dadurch gekennzeichnet, dass** nach dem Hydrolysieren der HMTBN und vor dem Neutralisieren eine Destillation bei einer Temperatur von 80 °C bis 120 °C und einem Druck von 0,5 bis 1,5 bar durchgeführt wird.

13. Verfahren nach Anspruch 1, **dadurch gekennzeichnet, dass** die erste Phase, die mindestens HMTBA und Salze umfasst, und die zweite Phase, die Salze enthält, durch Dekantieren separiert werden.

14. Verfahren nach einem der Ansprüche 1 bis 13, **dadurch gekennzeichnet, dass** die an HMTBA reiche Phase und/oder die an Salzen reiche Phase, die nach Abschluss der Chromatographie zurückgewonnen werden, verdampft werden und das recycelte resultierende wässrige Lösungsmittel bei mindestens einem der Schritte des Verfahrens.

## Claims

1. A method for manufacturing 2-hydroxy-4-methylthio-butyric acid (HMTBA) from 2-hydroxy-4-methylthio-butyronitrile (HMTBN), comprising the following steps:
hydrolyzing HMTBN to HMTBA in the presence of a mineral acid in an aqueous medium,
neutralizing said medium by adding a base,
separating a first phase comprising at least the HMTBA and salts and a second phase containing salts,
Said method being **characterized in that** the separation of the HMTBA from said salts of the first phase is carried out by subjecting the latter to chromatography.

2. The method according to claim 1, **characterized in that** the chromatography is carried out by treating the first phase containing the HMTBA on a resin.

3. The method according to claim 2, **characterized in that** the resin is an anionic resin which is preferably charged with anions selected from OH⁻, Cl⁻, SO₄²⁻.

4. The method according to claim 2, **characterized in that** the resin is a cationic resin which is preferably charged with cations selected from NH₄⁺, H⁺, Na⁺, K⁺ and Ca²⁺.

5. The method according to any one of the preceding claims, **characterized in that** the chromatography eluent is an aqueous solvent.

6. The method according to claim 5, **characterized in that** the aqueous solvent is selected from water and its mixtures with one or more organic solvents such as alcohols and in particular methanol and ethanol, furans and in particular tetrahydrofuran and acetonitrile.

7. The method according claim 5 or 6, **characterized in that** the aqueous solvent is selected from water, an acidic aqueous solution and a basic aqueous solution.

8. The method according to any one of the preceding claims, **characterized in that** the chromatography is carried out according to a simulated bed mode (SMB) or in a simulated sequential moving bed mode (SSMB).

9. The method according to any one of the preceding claims, **characterized in that**, before separation, the concentration of the first phase in HMTBA ranges from 30 to 90% (m/m), preferably 50 to 80% (m/m).

10. The method according to claim 9, **characterized in that** the HMTBA concentration is adjusted by adding water.

11. The method according to any one of the preceding claims, **characterized in that** the hydrolysis of the HMTBN is carried out in the presence of sulfuric acid and **in that** the neutralization is carried out by adding ammonia or ammonium hydroxide.

12. The method according to any one of the preceding claims, **characterized in that**, after the hydrolysis of the HMTBN and before the neutralization, a distillation is carried out at a temperature from 80 °C to 120 °C and a pressure from 0.5 to 1.5 bar.

13. The method of claim 1, **characterized in that** the first phase comprising at least the HMTBA and salts and the second phase containing salts, are separated by decantation.

14. The method according to any one of claims 1 to 13, **characterized in that** the phase rich in HMTBA and/or the phase rich in salts recovered at the end of the chromatography are subjected to evaporation and the resulting aqueous solvent is recycled to at least any one of the steps of said method.
